(19) European Patent Office

Europäisches Patentamt

Office européen des brevets

(11) EP 0 978 272 A1

(12) **EUROPEAN PATENT APPLICATION**

(43) Date of publication:
09.02.2000 Bulletin 2000/06

(51) Int. Cl.⁷: **A61K 7/06**

(21) Application number: **99115067.3**

(22) Date of filing: **05.08.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **07.08.1998 JP 22500198**

(71) Applicant: **Kao Corporation**
**Tokyo 103-8210 (JP)**

(72) Inventors:
• **Nagase, Shinobu,**
**c/o Kao Corporation**
**Tokyo 131-8501 (JP)**

• **Ando, Kenichi,**
**c/o Kao Corporation**
**Tokyo 131-8501 (JP)**
• **Kariya, Emiko,**
**c/o Kao Corporation**
**Tokyo 131-8501 (JP)**
• **Shibuichi, Satoshi,**
**c/o Kao Corporation**
**Tokyo 131-8501 (JP)**
• **Satoh, Naoki,**
**c/o Kao Corporation**
**Tokyo 131-8501 (JP)**

(74) Representative: **HOFFMANN - EITLE**
**Patent- und Rechtsanwälte**
**Arabellastrasse 4**
**81925 München (DE)**

(54) **Medulla care preparation**

(57) The invention relates to use of a compound having an amino group, hydroxyl group, carbonyl group or carboxyl group in its molecule and a molecular weight of 30 to 500 for the formulation of a medulla care preparation. The medulla care preparation directly or indirectly acts on medulla to improve the optical or mechanical properties of the hair.

EP 0 978 272 A1

## Description

TECHNICAL FIELD

The present invention relates to use of a compound for the formulation of a medulla care preparation which directly or indirectly acts on medulla present in the central part of hair to improve the optical or mechanical properties of the hair.

BACKGROUND ART

Various hair cosmetic compositions have heretofore been used for the purpose of improving the optical or mechanical properties of hair, such as gloss, softness and tightness or stiffness. However, these hair cosmetic compositions have been intended to act on the surface of the hair, cuticle present in the vicinity of the surface or cortex within the hair, and their effects have been temporary or insufficient.

The medulla present in the central part of hair has a porous structure that cavernous honeycomb cells are arranged in the axial direction thereof. Such a porous structure has raised a problem that light is irregularly reflected by the medulla to make a hair color dull looking or give the hair an appearance poor in a transparent feeling.

It is an object of the present invention to provide a preparation which acts on the medulla of the hair to exhibit an excellent effect of improving the optical or mechanical properties of the hair.

DISCLOSURE OF THE INVENTION

The present inventors have found that when a compound having specific functional group and molecular weight directly or indirectly acts on the medulla, the medulla can be taken care of.

According to the present invention, there is thus provided use of a compound having an amino group, hydroxyl group, carbonyl group or carboxyl group in its molecule and a molecular weight of 30 to 500 for the formulation of a medulla care preparation.

The term "medulla care" as used in the present invention means that scattering of light by the medulla is inhibited, and medullary lacunae are reduced.

The medulla care preparation according to the present invention can be used as an inhibitor against light scattering by the medulla, which can improve a dull looking hair color and an appearance poor in a, transparent feeling by inhibiting the light scattering by the medulla to impart a deep color, a transparent feeling and a bright gloss to the hair.

The medulla care preparation according to the present invention can also be used as an agent for reducing medullary lacunae, which can impart good softness and tightness or stiffness to the hair by reducing the medullary lacunae to improve the easy arrangement of the hair.

The medullar care preparation according to the present invention directly or indirectly acts on the medulla of the hair to cause a marked change in the porous structure of the medulla. The term "direct action" as used herein means that the medulla care preparation penetrates into medullary tissue to swell the fibers of the medullary tissue, and enters lactunary parts of the tissue to fill up lactunae. On the other hand, the term "indirect action" means that the medulla care preparation penetrates in the interior of the hair to compress medullary lactunae by the expansion of fibers about the medulla.

MODE FOR CARRYING OUT THE INVENTION

The compound useful in the practice of the present invention must have an amino group, hydroxyl group, carbonyl group or carboxyl group in its molecule. It is only necessary for the compound to have at least one of these groups. The compound may additionally have another group than these groups, for example, a mercapto group, guanidino group or the like. The compound preferably has a molecular weight of 30 to 500.

Examples of such a compound include α-hydroxy acids, β-hydroxy acids, 1,2-dicarboxylic acids, 1,3-dicarboxylic acids, aromatic carboxylic acids, amino acids, peptides, N-acylamino acids, urea, urea derivatives, guanidine, guanidine derivatives, primary amines, alcohols, aromatic alcohols and dialkyl ketones. Of these, the α-hydroxy acids, β-hydroxy acids, 1,2-dicarboxylic acids, 1,3-dicarboxylic acids, aromatic carboxylic acids, amino acids, urea, guanidine, aromatic alcohols and dialkyl ketones are particularly preferred.

Specific examples thereof include malic acid, succinic acid, maleic acid, salicylic acid, malonic acid, mandelic acid, lactic acid, glycolic acid, glycine, urea, phenylurea, citrulline, thiourea, guanidine, phenylguanidine, benzylguanidine, 3-methoxy-2-hydroxypropyl-guanidine, 2-(2-hydroxyethoxy)ethylguanidine, monoethanolamine, ethanol, n-propanol, benzyl alcohol, benzyloxyethanol, methyl salicylate, ethylene glycol salicylate and diethyl ketone.

Such compounds may be used either singly or in combination and are preferably incorporated in a proportion of 0.1 to 50 % by weight, more preferably 0.5 to 30 % by weight, particularly 1 to 20 % by weight based on the whole composition of the medulla care preparation because the effects can be achieved under milder treatment conditions.

In the medulla care preparations according to the present invention, ingredients commonly used in the classical hair cosmetic compositions, for example, cationic surfactant, anionic surfactants, nonionic surfactants, amphoteric surfactants, higher alcohols, acid dyes, oxidized dyes, silicones, cationic polymers, reducing agents, oxidizing agents, solvents, etc., may be suitably incorporated in addition to the above-described compounds so far as no detrimental influence is thereby imposed on the effects of the present invention.

The medulla care preparations according to the present invention can be formulated by mixing the individual components, and no particular limitation is imposed on the preparation form thereof. They may be provided in the form of, for example, liquid, gel, cream or aerosol.

The medulla care preparations according to the present invention are preferably kept at a pH of 2 to 12, particularly 2 to 6. In order to achieve the preferred pH, it is only necessary to adjust the pH of the preparation by adding an acid or alkali.

In the medulla care preparations according to the present invention, the sorbability on the hair, which is found in the following manner, is preferably 0.1 to 40 % by weight, particularly 1 to 40 % by weight. The sorbability on the hair is found by subjecting a hair bundle (10 g) to an immersion treatment with the medulla care preparation at 40°C for 60 minutes at a bath ratio (hair bundle:medulla care preparation) of 1:10 w/w. After the hair bundle after the treatment is washed with tap water, it is suspended in an environment of 20°C and 60% RH to leave it to stand for 24 hours, thereby measuring the weight of the hair bundle thus fully dried. The washing is conducted based on a method in which the hair bundle (10 g) is dipped for 1 minute in the tap water (1 liter) at 25°C and then pulled up while lightly shaking it. From the weight (W) of the hair bundle after treated and dried and the weight ($W_0$) of the hair bundle before the treatment, the sorbability on the hair is determined in accordance with the following equation:

$$\text{Sorbability on the hair (wt.\%)} = \frac{W - W_0}{W_0} \times 100$$

The medulla care preparation according to the present invention may be used by, for example, applying it to the hair to treat the hair at 15 to 70°C for 30 seconds to 12 hours. In this case, the medulla care preparation is preferably used at a bath ratio of the hair/the medulla care preparation being 1/0.01 to 1/10, particularly 1/0.05 to 1/2. Upon the treatment, an exothermic body warmer which makes use of, for example, the oxidation of iron powder, a heat accumulator which makes use of the heat capacity of a polymer having a molecular weight of at least 5,000, or the like may also be utilized as a heating material.

Whether the porous structure of the medulla has changed can be confirmed by, for example, an optical method. Since the porous structure of the medulla can be confirmed as a shining stripe in the central part of a hair by scattering light, it is only necessary to observe the proportion (change in the intensity of light scattering) of this shining stripe. More specifically, when a hair is lit up by a penlight through, for example, a simplified stereomicroscope, the medulla is clearly observed as a white stripe in the hair. As a quantifying method, it is only necessary to observe a proportion of the stripe to the overall length of the hair coming into view by a method such as visual observation or image processing. Even when the judgment is made by the visual observation, the quantity of the porous part of the medulla can be generally determined with a precision within plus or minus 10% or so.

Whether the medulla care preparation directly or indirectly has acted on the medulla can be judged from a change in the quantity of the porous part, i.e., a proportion of change in the intensity of light scattering by the medulla (rate of change in the length of the light stripe before and after the treatment). When a change of at least 20%, including an error in the measurement, based on the overall length of the hair coming into view is observed, the medulla care preparation may be generally judged to have acted on the medulla.

EXAMPLES

Example 1:

Their corresponding components shown in Tables 1 and 2 were mixed to formulate medulla care preparations, and the medulla care preparations thus obtained were used for hair to evaluate them as to the inhibitory effect on light scattering by medulla, and the appearance, softening/tightness or stiffness and easy arrangement of the hair. In each evaluation, the hair subjected to permanent treatment and bleaching treatment was used and treated with each preparation sample under conditions shown in Tables 1 and 2 to make its evaluation.

(Evaluation methods)

(1) Inhibitory effect on light scattering by medulla:

A simplified stereomicroscope [WIDE STAND MICRO, manufactured by PEAK CO., (10 magnifications)] was used to irradiate a hair with light from a direction inclined to the root of the hair (angle between a hair axis and an irradiation axis of light from a light source: 15 to 60 degrees), thereby observing the hair from a direction perpendicular to the hair axis on the same plane as the hair axis and the irradiation axis of the light. According to this method, regularly reflected light from the surface of the hair, which is an obstacle to the observation, can be removed, whereby only the porous structure at a medulla part is observed as a whitish stripe in the central part of the hair. The overall lengths of the medulla observed as the whitish stripe before and after the treatment were measured. The overall length before the treatment was regarded as 100%, thus evaluating how percent of the overall medulla length became unable to be observed after the treatment. The result was expressed in terms of a proportion (%) of the medulla that became unable to be observed.

(2) Appearance, softening/tightness or stiffness and easy arrangement of the hair:

A hair bundle 20 cm in length and 10 g in weight was treated with each preparation sample under conditions shown in Tables 1 and 2 to organoleptically evaluate the sample by 10 expert panelists in comparison with the hair bundle before the treatment in accordance with the following standard:

〈Appearance of hair〉

| | Score |
|---|---|
| The hair color becomes deeper, and the hair is overflowing with a transparent feeling and bright: | +1 |
| It is hard to say which they are: | 0 |
| The hair color becomes a dull looking, and the hair has no transparent feeling and is dry and loose: | -1. |

Each preparation sample was ranked by the total score of all the panelists as ◯ where the score was 4 points or higher, △ where the score was from 3 points to -3 points, or X where the score was -4 points or lower.

〈Softening/tightness stiffness〉

| | Score |
|---|---|
| The hair has a resilient feel and is tight and stiff: | +1 |
| It is hard to say which they are: | 0 |
| The hair has a soft feel and is supple: | -1. |

Each preparation sample was ranked by the total score of all the panelists as □ where the score was 4 points or higher, X where the score was from 3 points to -3 points, or ◯ where the score was -4 points or lower.

〈Easy arrangement of hair〉

|  | Score |
|---|---|
| The hair is well arranged without any stray hairs: | +1 |
| It is hard to say which they are: | 0 |
| The hair is not arranged due to many stray hairs: | -1. |

Each preparation sample was ranked by the total score of all the panelists as ◯ where the score was 4 points or higher, △ where the score was from 3 points to -3 points, or X where the score was -4 points or lower.

Table 1

| Component (wt.%) | Molecular weight | Before treatment | Invention product 1 | Invention product 2 | Invention product 3 | Invention product 4 | Invention product 5 |
|---|---|---|---|---|---|---|---|
| Malic acid | 134.09 | - | 4.0 | - | - | 4.0 | 4.0 |
| Succinic acid | 118.09 | - | - | 4.0 | - | - | - |
| Maleic acid | 116.07 | - | - | - | 4.0 | - | - |
| 2-Benzyloxyethanol | 152.19 | - | 10.0 | 10.0 | 10.0 | 10.0 | 10.0 |
| Ethanol | 46.07 | - | 15.0 | 15.0 | 15.0 | 15.0 | 15.0 |
| Glycine | 75.07 | - | - | - | - | 5.0 | - |
| Urea | 60.06 | - | - | - | - | - | 5.0 |
| pH adjuster (NaOH) | - | - | q.s. | q.s. | q.s. | q.s. | q.s. |
| Water | - | - | Balance | Balance | Balance | Balance | Balance |
| Bath ratio of hair to treatment agent | - | - | 1/10 | 1/10 | 1/10 | 1/10 | 1/10 |
| Treating temperature (°C) | - | - | 40 | 40 | 40 | 40 | 40 |
| Treating time (min) | - | - | 60 | 60 | 60 | 60 | 60 |
| pH | - | - | 3.0 | 3.0 | 3.0 | 3.0 | 3.0 |
| Sorbability on hair | - | 0% | 10% | 8% | 12% | 10% | 10% |
| Inhibitory effect on light scattering from medulla | - | 0% | 60% | 60% | 80% | 80% | 80% |
| Appearance of hair | - | × | ○ | ○ | ○ | ○ | ○ |
| Softening/tightness·stiffness | - | × | □ | □ | □ | ○ | ○ |
| Easy arrangement of hair | - | × | ○ | ○ | ○ | ○ | ○ |

Table 2

| Component (wt.%) | Molecular weight | Invention product 6 | Invention product 7 | Invention product 8 | Invention product 9 | Invention product 10 | Invention product 11 |
|---|---|---|---|---|---|---|---|
| Malic acid | 134.09 | 4.0 | - | - | 4.0 | 4.0 | 4.0 |
| Malonic acid | 104.06 | - | 4.0 | - | - | - | - |
| Salicylic acid | 138.12 | - | - | 1.0 | - | - | - |
| 2-Benzyloxyethanol | 152.19 | - | 10.0 | 10.0 | 10.0 | 10.0 | 10.0 |
| Benzyl alcohol | 108.14 | 8.0 | - | - | - | - | - |
| Ethanol | 46.07 | 30.0 | 15.0 | 15.0 | 15.0 | 15.0 | 15.0 |
| Methyl salicylate | 152.15 | - | - | - | 0.2 | - | - |
| Ethylene glycol salicylate | 182.17 | - | - | - | - | 0.2 | - |
| Monoethanolamine | 61.08 | - | - | - | - | - | 5.0 |
| pH adjuster (NaOH) | - | q.s. | q.s. | q.s. | q.s. | q.s. | q.s. |
| Water | - | Balance | Balance | Balance | Balance | Balance | Balance |
| Bath ratio of hair to treatment agent | - | 1/10 | 1/10 | 1/10 | 1/10 | 1/10 | 1/10 |
| Treating temperature (°C) | - | 40 | 40 | 40 | 40 | 40 | 40 |
| Treating time (min) | - | 60 | 60 | 60 | 60 | 60 | 60 |
| pH | - | 3.0 | 3.0 | 3.0 | 3.0 | 3.0 | 3.0 |
| Sorbability on hair | - | 7% | 9% | 7% | 8% | 10% | 8% |
| Inhibitory effect on light scattering from medulla | - | 60% | 60% | 70% | 80% | 80% | 80% |
| Appearance of hair | - | ○ | ○ | ○ | ○ | ○ | ○ |
| Softening/tightness·stiffness | - | □ | □ | □ | □ | □ | ○ |
| Easy arrangement of hair | - | ○ | ○ | ○ | ○ | ○ | ○ |

Example 2:

Medulla care preparations of the shampoo and rinse types having their corresponding compositions shown in Table 3 were respectively formulated. These preparations were used to conduct a shampooing/rinsing treatment 30 times repeatedly, thereby evaluating them as to the inhibitory effect on light scattering by medulla, and the appearance, softening/tightness or stiffness and easy arrangement of the hair in the same manner as in Example 1. The results are shown in Table 4.

Table 3

| Component (wt.%) | Molecular weight | Shampoo | Rinse |
|---|---|---|---|
| Lactic acid | 90.09 | 0.5 | - |
| Malic acid | 134.09 | 0.5 | 1.0 |
| 2-Benzyloxyethanol | 152.19 | 0.5 | 0.5 |
| Sodium dodecylpolyoxyethylene (2.0) sulfate | - | 5.0 | - |
| Cetyltrimethylammonium chloride | - | - | 0.8 |
| Dicetyldimethylammonium chloride | - | - | 0.5 |
| Cetylstearyl alcohol | - | - | 5.0 |
| Methylparaben | - | 0.1 | 0.1 |
| Perfume base | - | 0.5 | 0.5 |
| pH adjuster (NaOH) | - | q.s. | q.s. |
| Water | - | Balance | Balance |
| pH | - | 7.0 | 4.0 |
| Bath ratio of hair to treatment agent | - | 1/0.2 | 1/0.2 |
| Treating temperature (°C) | - | 30 | 30 |

Table 4

| | After shampooing and rinsing |
|---|---|
| Sorbability on hair | 2% |
| Inhibitory effect on light scattering by medulla | 60% |
| Appearance of hair | ○ |
| Softening/tightness stiffness | ○ |
| Easy arrangement of hair | ○ |

Example 3:

A hair manicure type medulla care preparation having a composition shown in Table 5 was formulated to evaluate it as to the inhibitory effect on light scattering by medulla, and the appearance, softening/tightness or stiffness and easy arrangement of the hair in the same manner as in Example 1. The results are shown collectively in Table 5.

Table 5

| Component (wt.%) | Molecular weight | Hair manicure type medulla care preparation |
|---|---|---|
| Malic acid | 134.09 | 4.0 |
| Glycolic acid | 76.05 | 2.0 |
| 2-Benzyloxyethanol | 152.19 | 10.0 |
| Ethanol | 46.07 | 10.0 |
| N-Methyl-2-pyrrolidone | 99.13 | 10.0 |
| Red Color No. 106 | - | 0.2 |
| Orange Color No. 205 | - | 0.8 |
| Hydroxyethyl cellulose | - | 2.0 |
| Methylparaben | - | 0.1 |
| Perfume base | - | 0.5 |
| pH adjuster (NaOH) | - | q.s. |
| Water | - | Balance |
| pH | - | 3.0 |
| Bath ratio of hair to treatment agent | - | 1/1 |
| Treating temperature (°C) | - | 30 |
| Treating time (min) | - | 30 |
| Sorbability on hair | 8% | |
| Inhibitory effect on light scattering by medulla | 70% | |
| Appearance of hair | ○ | |
| Softening/tightness stiffness | □ | |
| Easy arrangement of hair | ○ | |

## INDUSTRIAL APPLICABILITY

The medulla care preparations according to the present invention can directly or indirectly act on medulla to markedly improve the optical or mechanical properties of hair, and can impart a deep color, a transparent feeling and a bright gloss to the hair and moreover give good softness and tightness or stiffness to the hair to improve the easy arrangement of the hair.

## Claims

1.   Use of a compound having an amino group, hydroxyl group, carbonyl group or carboxyl group in its molecule and a molecular weight of 30 to 500 for the formulation of a medulla care preparation.

2.   The use according to Claim 1, wherein the medulla care preparation has a sorbability on hair of 0.1 to 40 % by weight when treating a hair bundle with the medulla care preparation at 40°C for 60 minutes at a bath ratio of 1:10 w/w.

3.   The use according to Claim 1 or 2, wherein the compound is selected from the group consisting of α-hydroxy acids, β-hydroxy acids, 1,2-dicarboxylic acids, 1,3-dicarboxylic acids, aromatic carboxylic acids, amino acids, peptides, N-acylamino acids, urea, urea derivatives, guanidine, guanidine derivatives, primary amines, alcohols, aromatic alcohols and dialkyl ketones.

4. The use according to any one of Claims 1 to 3, wherein the medulla care preparation inhibits scattering of light by medulla to take care of the medulla.

5. The use according to any one of Claims 1 to 3, wherein the medulla care preparation reduces medullary lacunae to take care of the medulla.

European Patent
Office

**EUROPEAN SEARCH REPORT**

Application Number

EP 99 11 5067

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 793 956 A (BEIERSDORF AG) 10 September 1997 (1997-09-10) * page 2, line 28 - line 31 * * page 10, line 44 - line 52 * * examples 1-4 * * claims 1-4 * | 1,3 | A61K7/06 |
| P,X | EP 0 858 794 A (KAO CORP) 19 August 1998 (1998-08-19) * page 2, line 5 - line 7; claims 1-13 * | 1,3 | |
| X | DE 195 33 211 A (GOLDWELL GMBH) 8 February 1996 (1996-02-08) * claims 1-4 * | 1,3 | |
| X | DE 195 04 914 C (GOLDWELL GMBH) 16 November 1995 (1995-11-16) * claims 1-4 * | 1,3 | |
| X | US 4 855 130 A (KONRAD EUGEN ET AL) 8 August 1989 (1989-08-08) * abstract * * claims 1-7 * | 1,3 | TECHNICAL FIELDS SEARCHED (Int.Cl.7)<br><br>A61K |
| X | PATENT ABSTRACTS OF JAPAN vol. 1998, no. 03, 27 February 1998 (1998-02-27) & JP 09 301831 A (HOYU CO LTD), 25 November 1997 (1997-11-25) * abstract * | 1,3 | |
| X | PATENT ABSTRACTS OF JAPAN vol. 1997, no. 08, 29 August 1997 (1997-08-29) & JP 09 100218 A (SHISEIDO CO LTD), 15 April 1997 (1997-04-15) * abstract * | 1,3 | |

The present search report has been drawn up for all claims

| Place of search | Date of completion of the search | Examiner |
|---|---|---|
| THE HAGUE | 12 November 1999 | Stienon, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 99 11 5067

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-1999

| Patent document cited in search report | | Publication date | Patent family member(s) | | Publication date |
|---|---|---|---|---|---|
| EP 0793956 | A | 10-09-1997 | DE | 19608775 A | 11-09-1997 |
| | | | JP | 9241135 A | 16-09-1997 |
| EP 0858794 | A | 19-08-1998 | JP | 10218738 A | 18-08-1998 |
| | | | CN | 1200921 A | 09-12-1998 |
| DE 19533211 | A | 08-02-1996 | CN | 1199332 A | 18-11-1998 |
| | | | GB | 2321595 A | 05-08-1998 |
| | | | WO | 9709029 A | 13-03-1997 |
| | | | JP | 9110650 A | 28-04-1997 |
| DE 19504914 | C | 16-11-1995 | AT | 155036 T | 15-07-1997 |
| | | | AU | 696033 B | 27-08-1998 |
| | | | AU | 4445796 A | 22-08-1996 |
| | | | CA | 2169530 A | 16-08-1996 |
| | | | DE | 59600010 D | 14-08-1997 |
| | | | DK | 727204 T | 02-02-1998 |
| | | | EP | 0727204 A | 21-08-1996 |
| | | | ES | 2105902 T | 16-10-1997 |
| | | | FI | 960654 A | 16-08-1996 |
| | | | JP | 8239312 A | 17-09-1996 |
| | | | US | 5785962 A | 28-07-1998 |
| US 4855130 | A | 08-08-1989 | DE | 3602746 A | 06-08-1987 |
| | | | AU | 595289 B | 29-03-1990 |
| | | | AU | 6896487 A | 25-08-1987 |
| | | | WO | 8704616 A | 13-08-1987 |
| | | | EP | 0234261 A | 02-09-1987 |
| JP 09301831 | A | 25-11-1997 | NONE | | |
| JP 09100218 | A | 15-04-1997 | NONE | | |

EPO FORM P0459

For more details about this annex : see Official Journal of the European Patent Office, No. 12/82